# EUROPEAN PATENT SPECIFICATION

(11) **EP 1 465 779 B1**
(45) Date of publication and mention of the grant of the patent: **28.05.2008**
(21) Application number: 02788239.8
(22) Date of filing: 20.12.2002
(51) Int. Cl.: G01N 21/87, G01N 33/38

(54) **ANTI-FRAUD METHOD FOR PROTECTING VALUABLE STONES**
BETRUGSVERHINDERUNGSVERFAHREN ZUM SCHUTZ VON WERTVOLLEN STEINEN
PROCEDE ANTI-FRAUDE DE PROTECTION DES PIERRES DE VALEUR

(30) Priority: 20.12.2001 GB 0130540
(43) Date of publication of application: 13.10.2004
(60) Divisional application: 07020709.7
(73) Proprietor: Boles, Julian, London EC1N 8NX (GB)
(72) Inventor: Boles, Julian, London EC1N 8NX (GB)
(74) Representative: Wotherspoon, Hugh Robert
(86) International application number: PCT/GB2002/005881
(87) International publication number: WO 2003/053715

(56) References cited:
- EP-A- 0 042 361
- EP-A- 0 256 196
- EP-A- 0 411 779
- WO-A-93/12496
- WO-A-99/61890
- US-A- 4 749 847
- US-A- 5 673 338
- US-A- 5 828 405
- US-A- 5 932 119
- US-A- 6 140 936

## Description

The present invention relates to a method for characterising precious or semi-precious stones.

It is conventional to characterise diamonds and other precious semi-precious stones by their carat (weight), colour, clarity and cut, known as the 4C's. These characteristics can be checked relatively easily by accurate weighing and optical examination and are conventionally recorded on a printed certificate which accompanies the gem and evidences its authenticity. However, the colour and clarity of a diamond are somewhat subjective and it is not unknown for fraudulent substitutions to be made in traded gems.

One approach to solving the above problems involves marking the gem and providing image data on a certificate - as disclosed in US 5,899,503.

Furthermore, a stolen diamond can effectively acquire a new identity by being re-cut, enabling it to be traded on the legitimate market with a low risk of detection and only a small loss in weight.

More generally, valuables such as gems, objets d'art and items of scientific value such as meteorites are not always easy to identify and are frequently the subject of fraudulent operations such as forgery or alteration or fraudulent transactions e.g. after being stolen by substitution for a similar item of higher value.

GB 2,358,541A discloses optical apparatus for capturing a three-dimensional image of a diamond. However the resulting digitised images are subject to degradation by pixellation and are limited in resolution.

An object of the present invention is to overcome or alleviate at least some of the above problems.

The invention provides a method of characterising a cut or uncut precious or semi-precious stone as defined by claim 1.

Preferably the image is an electron micrograph. The selected characteristic feature is an irregular region at the boundary of two or more facets, e.g. at the cutlet or (in the case of an emerald cut diamond) the keel which are highly characteristic of an individual gem stone, especially at the resolution of an electron microscope.

Preferably the digitised image is stored in an electronic tag. A particularly preferred electronic tag is the ME1 Hitachi-Maxell RFID chip as made by Hitachi. Such tags are highly miniaturised and can be written to and read from in a contactless manner and incorporated permanently in a certificate in a tamper-proof manner. For example, the tag can be sealed within a certificate of plastics material.

Other preferred features of this invention are defined in the dependent claims.

The irregular region is as described above, particularly the culet, keel, girdle, table or crown and preferably the electronic tag is an RFID (Radio Frequency Identification) tag.

Preferably the digitised image is an electron micrograph and preferably the digitised image is stored in encrypted form. Preferably the electronic tag is permanently secured to a certificate in a tamper-proof manner.

There is also disclosed an apparatus for certifying a valuable, the apparatus comprising means for acquiring a high (preferably higher-than-optical) resolution digitised image of at least one characterising region of the valuable, means for encrypting said digitised image and writing means arranged to write said encrypted digitised image to an electronic identifier tag associated with said valuable. Such apparatus can be used to provide genuine proof of both authenticity and ownership.

Preferably the apparatus further comprises transmitting means arranged to transmit said encrypted digitised image and an identifier for valuable to a remote server posting a database of such encrypted digitised images and identifiers.

Preferably the image acquiring means comprises an electron microscope

The method of the invention can be used for checking for fraudulent transactions involving or operations on a valuable, by reading a similar image from an electronic identifier tag associated with the valuable and comparing the images.

The method of the invention can be used together with the further step of receiving from a database of such images a previously acquired digitised image having an identifier corresponding to an identifier stored in said electronic identifier tag. Each identifier can be stored in association with its corresponding digitised image in the database and accordingly a digitised image can be retrieved by selecting the appropriate identifier.

The method of the invention can be used with financial transaction data pertaining to the valuable as identified by an identifier stored in said electronic identifier tag being received from a database. This database can either be the same database as the image database referred to above, or a completely separate database maintained by e.g. a financial institution such as a bank or a credit card or insurance company. This feature enables a register of transactions in a given valuable to be kept, so that any attempted transactions in a stolen valuable can be detected by comparison with this register.

The valuable is a cut or uncut precious or semi-precious stone, and the characterising region is an irregular region of the stone, e.g. the culet, keel, girdle, table or crown facets as noted above.

Preferably the digitised images are stored in encrypted form and are decrypted prior to the comparison.

Preferably the electronic tag is an RFID (Radio Frequency identification) tag, since such tags can be made in highly miniaturised form and hidden quite easily, particularly in larger valuables such as paintings or furniture.

Any copy of the valuable can easily be detected by scanning for the tag. Any tampering with the valuable can be detected by acquiring a fresh image of one or more characteristic regions and comparing these images with the corresponding stored images in the tag.

The stored image or images are of higher-than-optical resolution in order to discourage normal fraudulent alteration which is dependent upon the faker's eyesight with, at best, enhancement by optical means such as a magnifying glass. The acquired image need not be an electron micrograph.

In general, the advantages of the invention arise from the highly detailed information which can be stored securely, either in a very small electronic memory in an electronic tag associated with the cut or uncut precious or semi-precious stone (e.g. incorporated in a certificate associated with the valuable or even hidden in the valuable itself) or remotely e.g. in a database on a remote server. Preferably the image data is encrypted in order to provide further security.

Preferred embodiments of the invention are described below, by way of example only, with reference to Figures 1 to 4 of the accompanying drawings, wherein:
Figure 1 is a diagrammatic representation of apparatus for certifying or verifying a cut or uncut precious or semi-precious stone or other valuable;
Figure 2 is a diagrammatic perspective view showing a valuable having an identifier tag hidden thereon;
Figure 3 is an example of an electron micrograph of the culet of a diamond as obtained by the apparatus of Figure 1; and
Figure 4 is a block diagram illustrating a hardware and software system incorporating the apparatus of Figure 1.

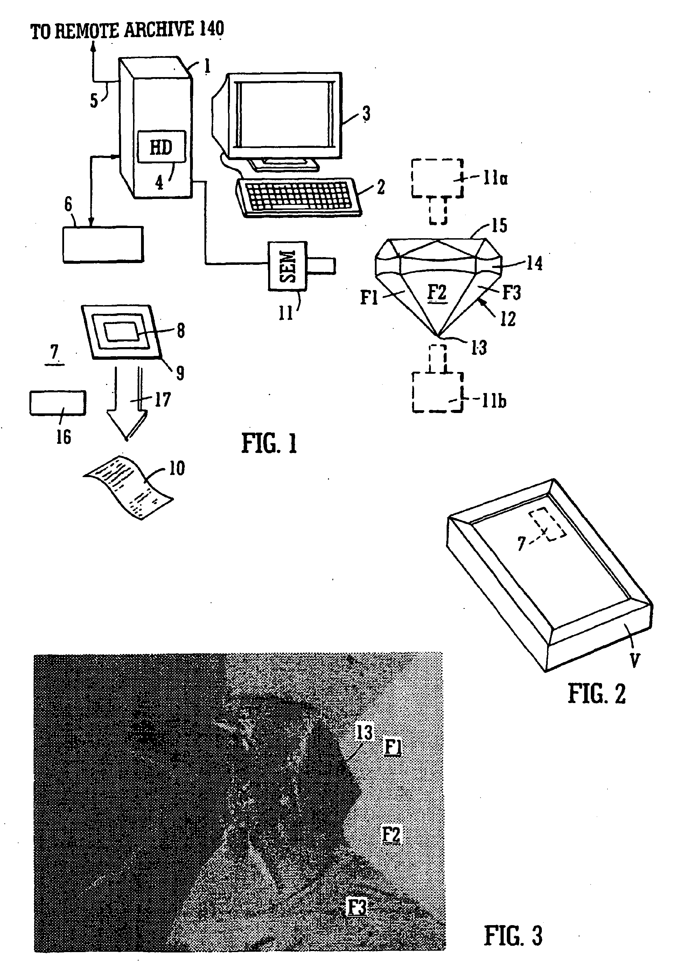

The apparatus shown in Figure 1 comprises a computer 1 having a hard disk 4 (and a conventional microprocessor provided with RAM and ROM and a conventional operating system) controlled from a keyboard 2 and optionally a pointing device such as a mousse (not shown). The computer 1 has a suitable interface (not shown) for the output of a scanning electron microscope 11 and images from the scanning electron microscope preferably with optical colour images can be displayed on a screen 3, stored on hard disk 4 and encrypted under the control of any suitable encryption software running on computer 1.

The resulting digitised images are output to an inductive writer 6 which is arranged to store the images in a memory 8 of a miniature RFID (Radio Frequency Identification) tag 7, such as Hitachi-Maxwell ME1 chip which picks up the wireless signals by means of a miniature on-chip 9. The tag typically has dimensions of 2.5mm x 2.5mm x 0.6mm.

The tag 7 now contains encrypted digitised electron micrograph images and preferably also optical images in non-volatile memory and can be read by reader/writer 6.

The tag is then processed in an encapsulation device 16 which, in a process indicated by arrow 17, embeds the tag in a certificate 10 which is suitably a plastic card on which can be printed a further data indicating e.g. the carat, colour, clarity and cut of the stone and one or more visual images of the stone. The images are provided with a unique identifier which is entered under keyboard control and transmitted from reader/write 6 to memory 8.

In Figure 1, a stone 12 is indicated on a greatly enlarged scale and it will be noted that the scanning electron microscope 11 is directed at a girdle region 14 or table region 15 which may or may not be cut or polished but which is highly characteristic of an individual gem stone in view of the large number of facet boundaries and minute irregularities it contains. The scanning electron microscope is also arranged to view the crown 15 of the stone 12 as indicated at 11 a and to view the culet 13 as indicated at 11 b. Encrypted digitised electron micrographs are acquired, preferably in at least these three views.

Referring to Figure 3, the culet 13 and the adjacent tips of facets F1, F2 and F3 are shown as they appear in the electron micrograph acquired by the scanning electron microscope in position 11b and the irregularities in this characteristic regions are readily apparent. The magnification is x 1000.

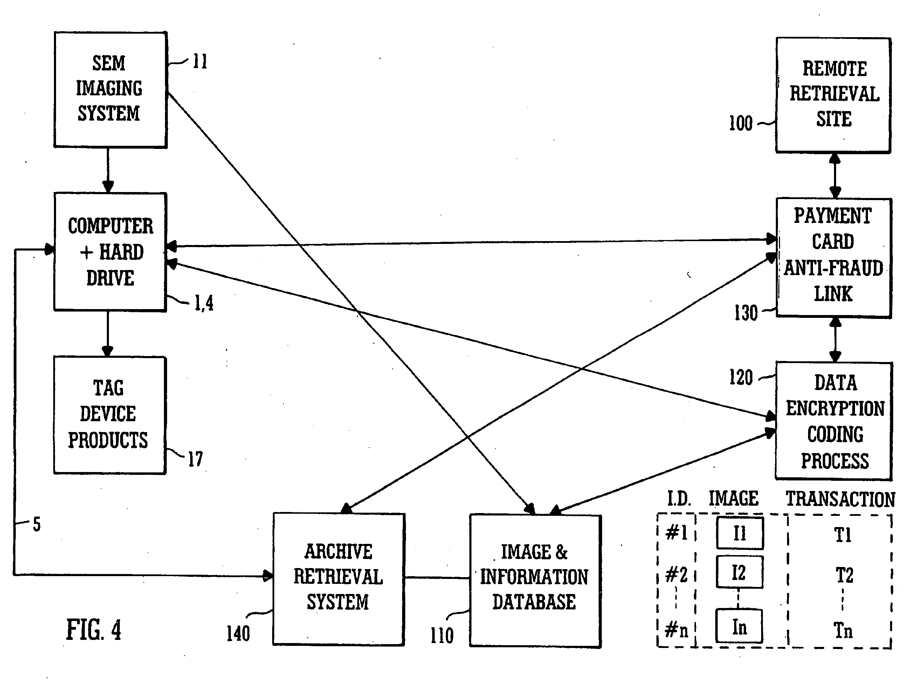

The apparatus of Figure 1 can also be used for verifying a diamond 12 or another gem stone or indeed any other valuable having an associated electronic tag containing digitised electron micrographs or other physical data of characteristic regions which can be used for identification purposes. These previously obtained electron micrographs are retrieved by computer 1 either directly from the associated RFID tag 7 using the reader/writer 6 or by accessing a remote archive 140 (Figure 4 - discussed in more detail below) using a communications link 5. These are displayed on the screen 3 and compared (e.g. visually) with electron micrographs acquired from corresponding view points from the gem stone 12 or other valuable as presented for evaluation. If the images match, then the gem stone or other valuable can be considered to be authentic. Provided that a suitable selection of images are compared, it is highly likely that any re-cutting or tampering with the stone will be detectable.

It should be noted that at an uncut extremity, a "natural" is often left at the culet 13 or at other regions of a gem stone to ensure that the weight just exceeds one of the standard weights, e.g. 1.00 carat. The value per carat of a gem stone falling fractionally below one of the standard carat weights is far less than the value per carat of a gem stone lying just above a standard carat. The "naturals" of a gem stone are normally highly irregular and therefore highly characteristic of an individual stone and are particularly suitable for imaging by an electron microscope for identification purposes. However, it is envisaged that other higher than optical resolution modes of imaging may be employed, e.g. X-ray, magnetic resonance imaging (MRI) or scanning tunnelling microscopy (STM).

In a variant of the method of certification illustrate in Figure 1, which is particularly applicable to larger valuables (V2) such as paintings, sculptures and furniture (but with increasing miniaturisation of RFID tags may also be applicable to smaller valuables including jewellery, even gem stones), the RFID tag 7 may be attached to the valuable V in a concealed fashion (e.g. in a rear corner of a painting as shown in Figure 2). One or more images of characteristic regions of the valuable V are stored in digitised encrypted form in the RFID tag 7 in a manner similar to that described with reference to Figure 1 and the valuable V can subsequently be authenticated by electronically scanning for the digitised image information stored in the RFID tag 7, retrieving and decrypting this information using the computer controlled reader/writer 6 of Figure 1 and comparing the digitised images with freshly acquired images of the same characteristic regions.

In a development of the arrangement shown in Figure 1, transaction data or other financial data pertaining to the valuable V or gem stone 12 can be stored in association with the digitised images in a remote database and retrieved by computer 1 using a communications link 5 in order to establish a complete chain of ownership back to the original producer of the gem stone 12 or, more generally, the first recorded owner of a valuable V.

Such a system is shown schematically in Figure 4. Referring to Figure 4, a scanning electron microscope imaging system 11 is linked to a hard drive 4 of a computer and is arranged to generate RFID tags storing encrypted digitised electron micrographs by process 17 as already described in relation to Figure 1. A communications link 5 links the computer with its hard drive to an archive retrieval system 140 (e.g. a server) which hosts an image and information database 110. As shown, this database includes fields for the identifier (# symbol 1 to # symbol n), images (I1, I2 .... In) and transactions (T1, T2 ..... Tn) such that inputting the identifier of a gem stone or other valuable enables the corresponding sets of images and transactions to be retrieved by computer 1.

The archive retrieval system 140 is also linked to a payment card anti-fraud link 130 which connects to a remote retrieval site 100 owned e.g. by a financial institution such as a credit card or insurance company or bank. Only if the gem stone 12 or valuable V is authenticated does the financial institution authorise a credit card or other payment from a buyer wishing to acquire the gem stone or other valuable from the purported owner. The payment card anti-fraud link 130 may also provide credit card verification or other credit information to computer 1 which may be located at a retail outlet.

Finally, data encryption and decryption may be provided by a separate block 120 which communicates with database 110, payment card anti-fraud link 130 and the computer 1.

## Claims

1. A method of characterising a cut or uncut precious or semi-precious stone (12) comprising the steps of acquiring a higher-than-optical resolution image (In) of at least one irregularity (13) at the boundary of two or more facets (F1, F2, F3) of a cut stone, said image being of resolution sufficiently high to define said at least one irregularity, said at least one irregularity being characteristic of the individual stone, digitising said image and storing the digitised image in association with an identifier (#n) of the stone.

2. A method according to claim 1 wherein said digitised image (In) is stored in an RFID (Radio Frequency Identification) tag (7).

3. A method according to claim 1 or claim 2 wherein said digitised image (In) is an electron micrograph.

4. A method according to any one of preceding claims 2-3 wherein said RFID tag (7) is permanently secured to an identification certificate (10) in a tamper-proof manner.

5. A method according to claim 4, wherein said RFID tag (7) is sealed within a certificate (10) of plastics material.

6. A method according to any one of preceding claims 2-5, wherein said digitised image (In) is stored in said RFID tag (7) in encrypted form.

7. A method according to any preceding claim wherein said digitised image (In) is stored remotely in an electronic database (110) so as to be retrievable therefrom using said identifier (#n).

8. A method according to claim 6, wherein said digitised image (In) is stored in said electronic database in association with financial transaction data pertaining to the stone (12).

## Patentansprüche

1. Verfahren zur Kennzeichnung eines geschliffenen oder ungeschliffenen Edel- oder Halbedelsteins (12), wobei das Verfahren die folgenden Schritte umfasst: das Erfassen eines Bils (ln) in höherer als der optischen Auflösung mindestens einer Unregelmäßigkeit (13) an der Begrenzung von zwei oder mehr Facetten (F1, F2, F3) eines geschliffenen Steins, wobei das genannte Bild eine ausreichend hohe Auflösung aufweist, um mindestens die genannte eine Unregelmäßigkeit zu definieren, wobei die genannte mindestens eine Unregelmäßigkeit kennzeichnend ist für den individuellen Stein; das Digitalisieren des genannten Bilds und das Speichern des digitalisierten Bilds in Verbindung mit einem Bezeichner (#n) des Steins.

2. Verfahren nach Anspruch 1, wobei das genannte digitalisierte Bild (In) in einem RFID (Radio Frequency Identification) Etikett (7) gespeichert wird.

3. Verfahren nach Anspruch 1 oder 2, wobei es sich bei dem genannten digitalisierten Bild (In) um ein Elektronenmikroskopbild handelt.

4. Verfahren nach einem der vorstehenden Ansprüche 2 und 3, wobei das genannte RFID Etikett (7) manipulationssicher dauerhaft an einem Identifikationszertifikat (10) angebracht wird.

5. Verfahren nach Anspruch 4, wobei das genannte RFID Etikett (70) in einem Zertifikat (10) aus einem Kunststoff versiegelt wird.

6. Verfahren nach einem der vorstehenden Ansprüche 2 bis 5, wobei das genannte digitalisierte Bild (In) in verschlüsselter Form in dem genannten RFID Etikett (7=) gespeichert wird.

7. Verfahren nach einem der vorstehenden Ansprüche, wobei das genannte digitalisierte Bild (In) entfernt in einer elektronischen Datenbank (110) gespeichert wird, so dass es aus dieser unter Verwendung des genannten Bezeichners (#n) abgerufen werden kann.

8. Verfahren nach Anspruch 6, wobei das genannte digitalisierte Bild (In) in der genannten elektronischen Datenbank in Verbindung mit dem Stein (12) betreffenden Finanztransaktionsdaten gespeichert wird.

## Revendications

1. Procédé de caractérisation d'une pierre taillée ou non taillée précieuse ou semi-précieuse (12) comprenant les étapes consistant à obtenir une image de résolution supérieure à la résolution optique (In) d'au moins une irrégularité (13) au niveau de la limite de deux ou plusieurs facettes (F1, F2, F3) d'une pierre taillée, ladite image étant d'une résolution suffisamment élevée pour définir ladite au moins une irrégularité, ladite au moins une irrégularité étant caractéristique de la pierre individuelle, à numériser ladite image et à stocker l'image numérisée en association avec un identificateur (#n) de la pierre.

2. Procédé selon la revendication 1 dans lequel ladite image numérisée (In) est stockée dans un indicateur RFID (Identification de fréquence radio) (7).

3. Procédé selon la revendication 1 ou la revendication 2 dans lequel ladite image numérisée (In) est un micrographe d'électron.

4. Procédé selon l'une quelconque des revendications précédentes 2 et 3 dans lequel ledit indicateur RFID (7) est associé de manière permanente avec un certificat d'identification (10) d'une manière anti-fraude.

5. Procédé selon la revendication 4, dans lequel ledit indicateur RFID (7) est scellé à l'intérieur d'un certificat (10) de matériau plastique.

6. Procédé selon l'une quelconque des revendications précédentes 2 à 5, dans lequel ladite image numérisée (In) est stockée dans ledit indicateur RFID (7) sous forme chiffrée.

7. Procédé selon l'une quelconque des revendications précédentes dans lequel ladite image numérisée (In) est stockée à distance dans une base de données électronique (110) de manière à pouvoir être récupérée dans celle-ci en utilisant ledit identificateur (#n).

8. Procédé selon la revendication 6, dans lequel ladite image numérisée (In) est stockée dans ladite base de données électronique en association avec des données de transaction financière concernant la pierre (12).
